# EUROPEAN PATENT SPECIFICATION

(11) **EP 1 565 754 B1**
(45) Date of publication and mention of the grant of the patent: **01.08.2007**
(21) Application number: 03811464.1
(22) Date of filing: 27.10.2003
(51) Int. Cl.: G01N 33/94, A61K 47/48, C07G 11/00, C07K 14/00, G01N 33/58, G01N 33/533

(54) **NEW USES OF PROTEINS ENCODED BY BLE GENES AND ANTIBIOTICS FROM THE BLEOMYCIN FAMILY**
NEUARTIGE VERWENDUNGEN VON DURCH BLE-GENE KODIERTEN PROTEINEN UND ANTIBIOTIKA AUS DER BLEOMYCIN-FAMILIE
NOUVELLES UTILISATIONS DE PROTEINES CODEES PAR DES GENES BLE ET ANTIBIOTIQUES DE LA FAMILLE BLEOMYCINE

(30) Priority: 25.10.2002 GB 0224872; 20.12.2002 GB 0229640; 31.03.2003 GB 0307379
(43) Date of publication of application: 24.08.2005
(73) Proprietor: SENSE PROTEOMIC LIMITED, Maidenhead, Berkshire SL6 7RJ (GB)
(72) Inventor: Hart, Darren, Sense Therapeutic Limited, Maidenhead, Berkshire SL6 7RJ (GB); Godber, Ben, Sense Therapeutic Limited, Maidenhead, Berkshire SL6 7RJ (GB); Blackburn, Jonathan M., Sense Therapeutic Limited, Maidenhead, Berkshire SL6 7RJ (GB); McAndrews, Mike, Sense Therapeutic Limited, Maidenhead, Berkshire SL6 7RJ (GB)
(74) Representative: Peter, Beate
(86) International application number: PCT/IB2003/005430
(87) International publication number: WO 2004/046730

(56) References cited:
- WO-A-02/27327
- US-A- 4 705 616
- US-A- 5 420 228
- US-A- 5 453 614
- US-A- 6 027 900
- US-B1- 2003 022 151
- BENNETT R P ET AL: "Fusion of green fluorescent protein with the Zeocin-resistance marker allows visual screening and drug selection of transfected eukaryotic cells" BIOTECHNIQUES, EATON PUBLISHING, NATICK, US, vol. 24, no. 3, March 1998 (1998-03), pages 478-482, XP002114401 ISSN: 0736-6205
- CALMELS THIERRY P G ET AL: "Nuclear localization of bacterial Streptoalloteichus hindustanus bleomycin resistance protein in mammalian cells" MOLECULAR PHARMACOLOGY, vol. 44, no. 6, 1993, pages 1135-1141, XP008030691 ISSN: 0026-895X
- Cayla catalogue, zeocin
- Oxford dictionary definition of "medium"
- GE Healthcare company protocols

## Description

### FIELD OF THE INVENTION

The present invention relates to new uses for the family of "ble" genes, for example Sh ble, Tn5 ble and Sa ble, and the family of proteins expressed by these genes which are able to reversibly bind to the bleomycin family of antibiotics. The bleomycin family of antibiotics are DNA- cleaving glycopeptides and include bleomycin, phleomycin, tallysomycin, pepleomycin and Phleomycin D1. The invention also relates to new uses of these antibiotics. More particularly it relates to fusion proteins comprising able protein and tools, methods and products which depend on the specificity between the ble protein and the antibiotics of the bleomycin family.

When these ble genes are expressed together with another protein as fusion proteins or the ble gene products are otherwise fused or linked to other molecules, particularly proteins, they can, for example, be used to strongly or weakly bind the fusion protein to a surface, such as an array, particular a microarray, a glass slide or a microtitre plate (where strong binding is generally desired) as well as to, for example, beads or other similar forms which are used in affinity purification (where weak binding is generally desired).

The invention however relates not only to the use of this pairing in binding molecules to surfaces, particularly though not essentially arrayable surfaces, but also to the use of able fusion protein additionally as a folding and solubility marker. It also relates to the use of "labelled" antibiotics from the bleomycin family which due to their specificity to the ble proteins make them useful as markers of cellular localisation.

### BACKGROUND OF THE INVENTION

Expression of human proteins in heterologous systems such as bacteria, yeast, insect cells or mammalian cells can result in the production of incorrectly folded proteins resulting in the formation of insoluble aggregates and / or a low yield of expressed proteins. For all functional proteomic work the production of correctly folded or native proteins is essential and a great deal of work is often performed to optimise the expression of individual proteins.

Two areas where incorrect folding and poor solubility may be significant are set out below:

### Problematic insolubility of engineered proteins

The manipulation of protein function by genetic engineering is a major field of study with strong commercial and academic drivers. The desired characteristics sought from such engineered proteins include altered substrate specificity, novel catalytic function, improved binding specificity and improved thermal or pH tolerance. However, the primary mutations introduced into the protein frequently result in loss of soluble expression and compensating mutations to restore solubility are usually impossible to predict.

### Problematic insolubility of proteins expressed recombinantly in heterologous hosts

Expressing recombinant proteins in *Escherichia coli* is often preferred, especially for bulk protein production (e.g. industrial enzymes, for X-ray crystallography), due to the ease of fermentation, absence of heterogeneous post-translational modification, simplicity of subsequent processing steps and high yields of material. However a frequent problem encountered, especially with eukaryotic proteins, is that the recombinant material is expressed insolubly and is therefore inactive. Such problematic proteins may be expressed solubly in more complicated systems (e.g. *Pichia pastoris,* baculovirus), but the lower yields and more expensive process can result in a commercially less attractive product.

In both of these cases, a method for generating soluble forms of an insoluble protein has great utility. Random mutagenesis of the protein to generate a library of variants followed by selection or screening for soluble expression is one approach. This is often referred to as "directed evolution". The requirements of such a screen are that many variant clones can be assessed in parallel since the libraries involved are large (often >10⁶ members). In some cases the activity of the target protein may be assayed directly. However, this is relatively rare and often requires each clone to be lysed in the wells of a microtitre plate and often the proteins have no assayable activity.

A more efficient method is to fuse to the protein a second "reporter" protein with a visually assayable phenotype. Since the two proteins are physically linked, the solubility of the reporter (and therefore presence of the observable phenotype) is dependent on the solubility of the other. Several such reporter systems have been reported. Preferably the phenotype can be observed with no process steps (i.e. when clones are still growing as colonies on agar plates) permitting a high throughput analysis.

Large scale expression of recombinant proteins for the manufacture of protein arrays is one area of proteomics which involves the functional characterisation of proteins in a parallel manner. Many thousands of proteins are required to be expressed in a soluble and folded manner. Expression libraries are usually employed whereby genes are placed under the control of a promoter and expression of the gene is then induced. The next step is to assess which clones express folded recombinant protein. On an individual basis, this is usually achieved by fractionating cell lysates into soluble and insoluble components by centrifugation and subsequent analysis of the fractions by gel electrophoresis. This approach is very low throughput and scale up to the level required to screen libraries is logistically infeasible. Thus, a need exists for a method to screen expression libraries for clones that produce soluble, folded proteins that may then be used for functional studies. Fusion of the recombinant proteins to a reporter protein permits simple determination of its solubility.

Before considering what has been done to date it is necessary to make the important distinction between a "screen" in which all members of a collection are assessed with a subset conforming to the desired characteristics being isolated, and a "selection" in which only the members of interest are observable.

Selections are more powerful than screens when dealing with very large numbers since practical limitations exists associated with handling and analysing large numbers of clones. Common examples of screens used in molecular biology are blue/white selection and GFP fluorescence.

Solubility/ folding reporters resulting in a colour based (visual) screen that have been described use β-galactosidase (Wigley, W.C., Stidham, R.D., Smith, N.M., Hunt, J.F. & Thomas, P.J. Protein solubility and folding monitored in vivo by structural complementation of a genetic marker protein. Nat. Biotechnol. 19, 131-135 (2001); and green fluorescent protein (GFP). (Waldo, G.S., Standish, B.M., Berendzen, J. & Terwilliger, T.C. Rapid protein-folding assay using green fluorescent protein. Nat. Biotechnol. 17, 691-695 (1999).)

Optimisation of protein solubility by directed evolution for use in structural studies has also been performed using GFP. (Pédelacq, J.D. et al. Engineering soluble proteins for structural genomics. Nat. Biotechnol. 20, 927-932 (2002).)

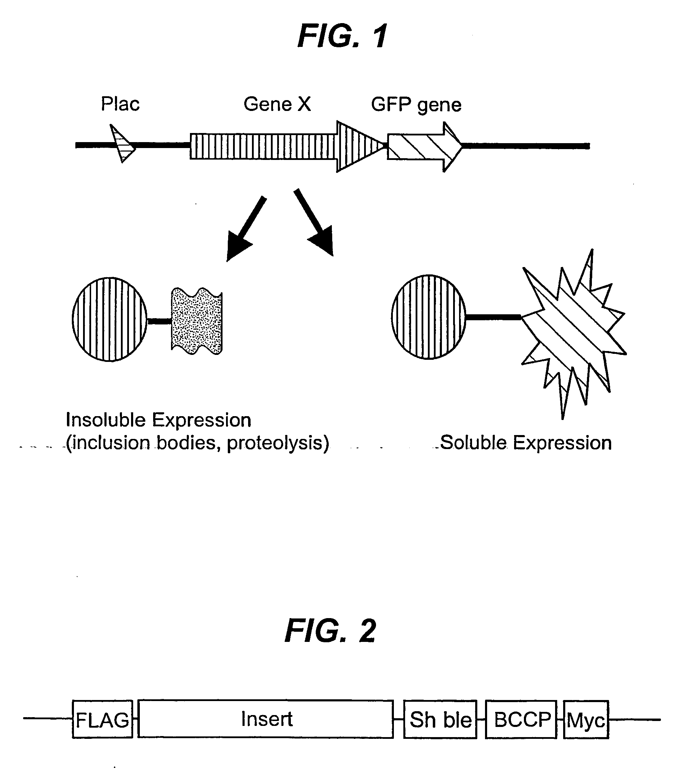

Several examples of exploiting fusion markers as indicators of protein folding and solubility exist in the literature. The principle that underlies these systems is the observation that protein folding and solubility are closely correlated since misfolded protein usually forms insoluble aggregates or are heavily proteolysed by the host cell. It is therefore assumed that if a protein is solubly expressed in an unproteolysed form, it is in its correctly folded form. If a fusion is made between one protein and another, the folding and solubility of one domain is linked to that of the other. This is shown in Fig 1 which illustrates the principle of a folding marker for assessing solubility of the gene X expression product. Only when the protein product of gene X is soluble is the phenotype of the fusion apparent. In this case, the fusion results in green fluorescent colonies that can be clearly identified.

A life-or-death selection for solubility and folding has been described employing chloramphenicol acetyl transferase (CAT) (Maxwell, K.L., Mittermaier, A.K., Forman-Kay, J.D. & Davidson, A.R. A simple in vivo assay for increased protein solubility. Protein Sci. 8, 1908-1911 (1999).)

Screening methods involving generation of a chimeric protein comprising a region of an unstable protein linked to a region of a marker gene product were reported in US patent application US 2003/0022151.

### DEFINITIONS

As defined herein "ble" genes are a family of genes which express proteins which reversibly bind the glycopeptide antibiotics of the bleomycin family, and include, but are not limited to Sh ble, Tn5 ble and Sa ble. In general these genes (or more precisely the gene products encoded by these genes) confer to their host resistance to the glycopeptide antibiotics of the bleomycin family,

The "bleomycin family of antibiotics" are DNA - cleaving glycopeptides and include, but are not limited to, bleomycin, phleomycin, tallysomycin, pepleomycin and Phleomycin D1.

The term "proteins", as used herein, is used to include both whole proteins, polypeptides, and sub units or domains thereof.

A "fusion protein", as used herein, refers to a protein, which comprises a "tag" at the N and/ or C terminus which binds to members of the bleomycin family of antibiotics. The fusion protein may be expressed as a fusion protein from a ble gene containing genetic construct or may be formed by either intein mediated splicing of, for example, two separate polypeptides or by the chemical ligation of two such peptides according to methods known in the art.

The "tag" is then used as a signalling component and/ or a means to manipulate the fusion protein further. Thus, it may be used to bind the fusion protein to a surface or to another molecule, such as for example a visual indicator molecule, such as a fluorescent molecule, or to indicate a characteristic of the fusion protein e.g. that it is folded such that it is likely to be functional.

The relative terms "strong binder" and "weak binder" and "high density " and "low density" are used herein to functionally distinguish between examples where it is desirable to achieve a substantially irreversible bond and the situation where it is intended that binding will be reversed such as in affinity purification. Thus, because for example the Sh ble protein is a dimer that can bind two molecules of bleomycin co-operatively (the first affinity is ~600nM whilst the second affinity is ~100nM) it is possible to use the ble proteins binding characteristics in a selective manner. For example, if one has a surface (e.g. a bead) coated with bleomycin at low density, then one would expect on average that one Sh ble dimer would be bound by one bleomycin molecule. In contrast, if one has a surface (e.g. a bead) coated with bleomycin at a high density, then one would expect on average that one Sh ble dimer would be bound by two bleomycin molecules (each binding one of the two dimer subunits), particularly if the bleomycin molecules were themselves attached to flexible linkers (e.g. polyethyleneglycol (PEG) polymers). Since in this latter case the two bleomycin molecules are effectively linked together via the surface, the second binding affinity will now be several orders of magnitude greater than where the two molecules are not linked together. In other words, the binding affinity will be significantly enhanced by a chelate effect. This effect can be utilised to advantage since one would want different binding affinities in, for example, an array (where one wants a substantially irreversible binding) and a reversible capture system such as a bead.

So, by controlling the surface density of the antibiotic, such as, bleomycin, it should be possible to readily control whether the Sh ble fusions bind weakly or strongly to the surface. Weak binding would be appropriate for affinity purifications whilst strong binding would be appropriate for immobilisation into arrays.

As an alternative to immobilising the bleomycin molecules onto a surface, one can covalently couple them to, for example amine-reactive fluorescent dyes, such as NHS-activated fluorescein, Cy3, Cy5, and Rhodamine. It should then be possible to diffuse the fluorescent bleomycin derivatives into whole cells (both prokaryotic and eukaryotic cells), whereupon the derivatives would bind selectively to Sh ble fusion proteins. In this way the Sh ble tag can be used as a marker of cellular localisation (in the same way as GFP is currently used). This will also enable the Sh ble tag to be used as both a selectable (i.e. life-or-death) marker and a screenable (i.e. fluorescence-based) marker.

In one aspect of the invention the support takes the form of a probe which is capable of acting as a target in analysis by laser desorption/ionisation mass spectrometry, for example, matrix assisted laser desorption/ionisation (MALDI). The probe carries the analytes, for example proteins, during such processes and interacts with the repeller lens of the ion-optic assembly found in laser desorption/ionisation time-of-flight (TOF) mass spectrometers of the art, such that the analytes are converted to gaseous ions to permit analysis. For example, the probes of the invention may be derived from targets for MALDI analysis as known in the art, which are treated such that a high affinity protein binding moiety e.g. Phleomycin D1 are present on the probe surface which bind Sh ble fusion proteins for subsequent analysis. For example, conventional glass or gold MALDI targets may be used.

As defined herein a "micro array" is an array where the size of the discrete target areas i.e. the individual areas probed by a laser, is in the order of micrometers or less. Whilst at the upper end of the scale, around 1000 micrometers diameter, they may be visible to the naked eye, at the lower end of the scale the discrete target areas will not be clearly distinguished by the naked eye.

The "arrays" will typically be arranged in matrices comprising several rows and columns. The number of discrete target areas will depend upon what is being screened though it is generally desirable to have a high density of these discrete areas on the probe surface as this will facilitate high through put screening. Typically a probe will comprise at least 10, more preferably at least 100, more preferably at least 1000 and as many as 10,000 or more target areas produced thereon. (Typically a probe surface will have an area of around 10,000mm² - a Bruker probe has an area of 10292mm² although there is no requirement to use the whole of the probe and the microarray can be applied in one or more matrices thereon.) The actual density in a given matrices will depend upon the size of the discrete target area (which will typically be printed as a spot) and the spacing between adjacent spots. Thus the discrete target areas will typically be present at a density of greater than 1 discrete target areas per mm² within any matrices.

"Linker molecules" are molecules which function as their name suggests. They are molecules comprising functional groups which allow bridges to be formed between different molecules.

### SUMMARY OF THE INVENTION

The protein product of, for example, the S*h ble* gene encodes a protein that when expressed by a cell, confers resistance to antibiotics of the bleomycin family including phleomycin D1 by binding to the antibiotic and sequestering it (Gatignol, A., Durand, H- & Tiraby, G. Bleomycin resistance conferred by a drug-binding protein. FEBS Lett 230, 171-175. (1988)).

These antibiotics otherwise induce DNA strand breakage. When, for example, the *Sh ble* gene is fused in tandem with a second gene of interest, thereby encoding a fusion protein, expression of the fusion protein also confers resistance to Phleomycin D1. If the gene of interest encodes an insoluble protein or protein fragment, the Phleomycin D1 resistance-conferring phenotype is not observed since the fusion protein is insoluble and incapable of binding the antibiotic. This forms the basis of a novel life-or-death selection for folded, soluble proteins.

Additionally, since, for example, the Sh ble protein binds Phleomycin D1 tightly and co-operatively the inventors have shown that it can be used as an affinity tag if a surface is provided that is derivatised with, for example, Phleomycin D1 or an analogue.

Furthemore, by, for example, linking a visual indicator to the antibiotic and allowing the labelled antibiotic to bind the fusion protein via the tag it should be possible to use the ble fusion protein as, for example, a marker for cellular localisation.

According to a first aspect of the present invention there is provided a method of immobilising a protein to a surface wherein the protein is provided to the surface as a ble fusion protein and the surface is a surface derivatised with an antibiotic from the bleomycin family.

In a preferred embodiment the ble fusion protein is used as an expression and folding marker and an affinity tag.

Antibiotics from the bleomycin family include, but are not limited to bleomycin, phleomycin, tallysomycin, pepleomycin and Phleomycin D1. Preferred members include bleomycin A2, bleomycin A5, bleomycin A6, bleomycin B2 and Phleomycin D1.

An advantage with bleomycin A5 and A6 is that they comprise a terminal primary amine group which can be used to couple the protein to, for example, an amine reactive surface. Typical groups used to produce an amine reactive surface include, but are not limited to: aldehydes, epoxides, N-hydroxysuccinamides, and isothionates. Of course other functional groups present on the antibiotics may be used to couple the antibiotics to a surface, and this will be apparent to the person skilled in the art.

In a preferred example coupling may be achieved by reacting the amine group with an amine reactive surface, such as, for example, a NHS activated, polyethyleneglycol (PEG) derivitized surface.

In a preferred embodiment the surface is the surface of an array, more particularly a microarray, and more particularly still a MALDI array although it could be a microtitre plate, a glass slide, (the surface being the surface of a probe) or a bead, (the surface being that of a purification media).

Thus according to a second aspect of the present invention there is provided a probe characterised in that it has a target surface comprising an array having a plurality of discrete target areas presenting one or more analyte capture moieties comprising an antibiotic from the bleomycin family.

Such a target surface typically has a high density coating of the antibiotic such that the target analyte is captured with a high affinity, preferably less than 400nM, more preferably less than 200nM, more particularly still less 150nM. In the specific example noted the Sh ble has an affinity in the order of 100nM or less when two molecules of bleomycin are bound.

According to a third aspect of the present invention there is provided a purification medium comprising a target surface presenting one or more analyte capture moieties comprising an antibiotic from the bleomycin family.

Such a target surface typically has a low density coating of the antibiotic such that the target analyte is captured with a low affinity, typically in the order of greater than 400nM, and more preferably greater than 500nM. In the specific example noted the Sh ble has an affinity in the order of 600nM when a single molecule of bleomycin is bound. Ideally the affinity would be in the µM range but sufficient that the proteins are bound.

In either case the antibiotic may be linked to the surface via a flexible linker such as an activated polyethyleneglycol (PEG).

According to a fourth aspect of the present invention there is provided an antibiotic from the bleomycin family characterised in that it is tagged with a marker.

Preferably the marker is a visual marker such as, for example, a fluorescent marker. Alternatively it could be labelled with a radioactive isotope. Preferred fluorescent markers include, but are not limited to NHS activated fluorescein, Cy3, Cy5 and Rhodamine.

A tool for the production of the fusion proteins is a vector consisting essentially of a ble gene downstream of a linker DNA which in use is excised and replaced with a DNA encoding a protein to be expressed as a ble fusion protein.

In one embodiment the ble vector is provided as one of the components of a kit for making an array along with a surface which has been derivatised with an antibiotic from the bleomycin family or the components for making such a derivatised surface.

According to a fifth aspect of the present invention there is provided a method for generating soluble forms of an insoluble protein comprising:
i) generating a library of protein variants; and
ii) selecting colonies for the presence of a soluble protein by expressing the protein as a ble fusion protein and selecting on an antibiotic from the bleomycin family.
iii) growing up the selected colonies in liquid culture, lysing and printing a crude lysate onto a surface derivatised with an antibiotic from the bleomycin family, preferably the method further comprises washing the tagged surface to remove non-bound materials.

A previous life-or-death selection utilising chloramphenicol acetyl transferase (CAT) is known, but is not very effective (Maxwell, K.L., Mittermaier, A.K., Forman-Kay, J.D. & Davidson, A.R A simple in vivo assay for increased protein solubility. Protein Sci. 8,1908-1911 (1999).) due to its narrow window of toxicity. i.e. the concentration of antibiotic that kills cells with no CAT is close to that which permits survival of cells with CAT. Also, it does not directly enable the subsequent downstream purification or immobilisation of the expressed protein since CAT enzymatically destroys the antibiotic and does not bind the product with significant affinity.

According to an sixth aspect of the present invention there is provided a method of purifying a Sh ble fusion protein from a crude extract comprising the step of immobilising it on a surface via an antibiotic from the bleomycin family and optionally releasing it there from.

According to a seventh aspect of the invention there is provided a method of identifying the cellular localisation of a protein comprising
i) expressing the protein as a ble fusion protein in a cell,
ii) introducing a labelled antibiotic from the bleomycin family into the cell, and detecting the labelled antibiotic.

The method may be qualitive or quantitive in which case the method comprises a further step of quantitating the labelled antibiotic. This may be done by measuring the intensity of fluorescence where the labelled antibiotic is fluorescently labelled.

### BRIEF DESCRIPTION OF THE DRAWINGS

The various aspects of the invention are further described by way of example only with reference to the following Figs and Examples in which:
Fig 1 is a schematic demonstrating the principle of using a folding marker for assessing the solubility of a "gene X" expression product. Only when the protein product of gene X is soluble is the phenotype of the fusion apparent. In this case, the fusion results in green fluorescent colonies that can be clearly identified;
Fig 2 is a vector according to one aspect of the invention incorporating a Sh ble gene;
Fig 3a and b illustrate the purification of a Sh ble fusion protein on Bleomycin Sepharose
   A) is a Coomassie stained gel, and
   B) is a Streptavidin HRP probed western.
   Lanes 1-6 are loaded as follows:
   1) lysate,
   2) Bleomycin-Sepharose after incubation with lysate,
   3) unbound lysate after incubation with Bleomycin-Sepharose,
   4) Bleomycin elution,
   5) Bleomycin-Sepharose after Bleomycin elution, and
   6) unbound lysate after incubation with Bleomycin-Sepharose.

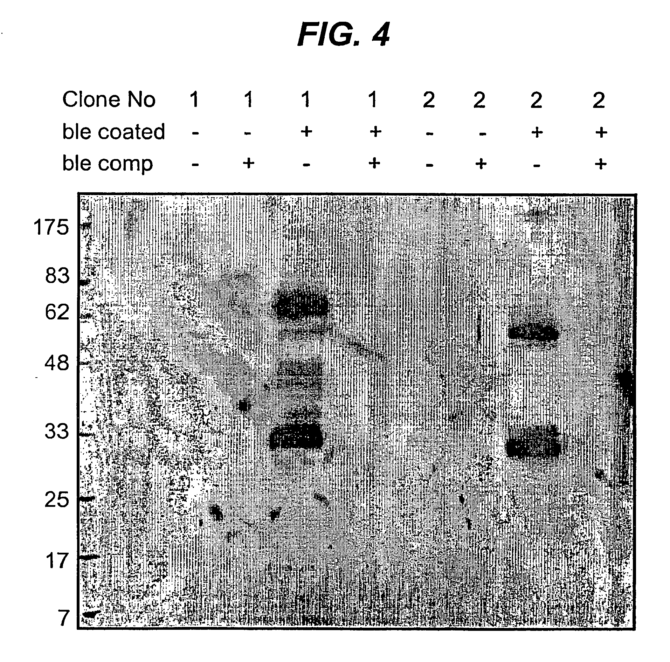

Fig 4 illustrates the immobilisation of Sh Ble fusion proteins on a Bleomycin derivatised microtitre plate. Lysates of two different Sh ble fusion protein clones were added to wells of Bleomycin derivatised and control microtitre plate in the presence and absence of competing Bleomycin;

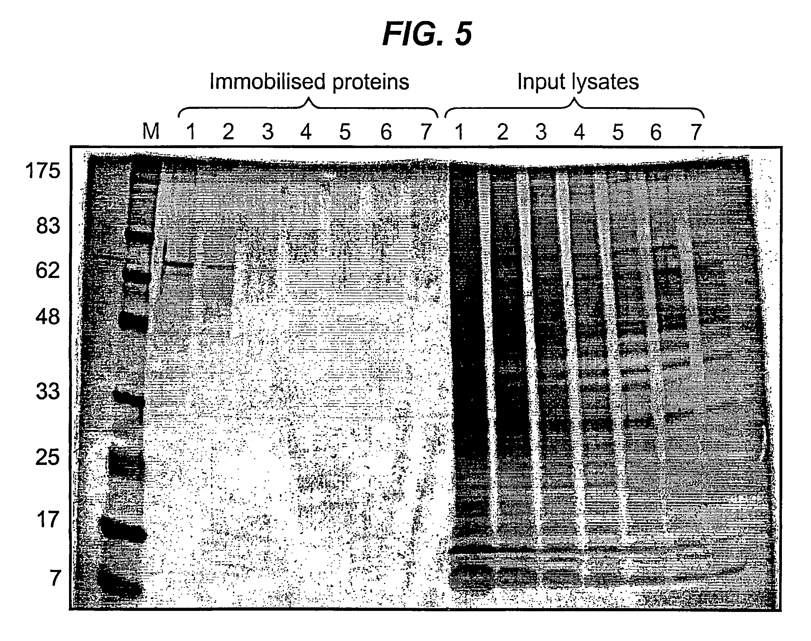

Fig 5 illustrates the purification of immobilised Sh ble fusion proteins on Bleomycin coated microwells; (Sh ble fusion protein captured from lysates on Bleomycin derivatised microwells was run on SDS PAGE. Input lysates were also run for comparison. Input lysates were a 2 fold dilution series, with lane 1 representing undiluted lysate and lane 7 representing a 1 in 64 dilution.)

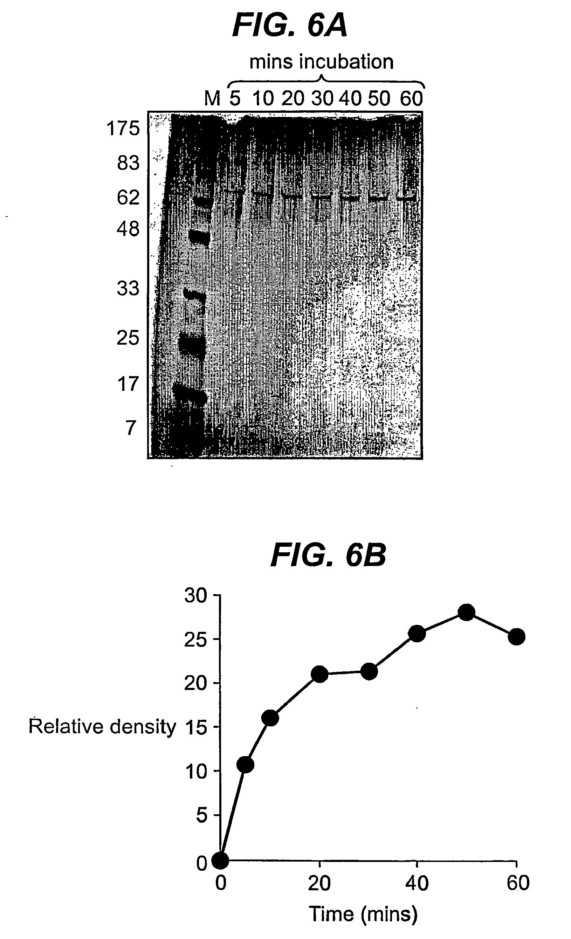

Fig 6 shows the rate of immobilization of Sh ble fusion protein on Bleomycin derivatised microwell; (Cleared lysates of Sh ble fusion protein clone 1 were incubated for varying times in a Bleomycin derivatised microwell. A) Silver stained SDS PAGE showing amount of fusion protein immobilized. B) The bands were quantified and plotted against time.)

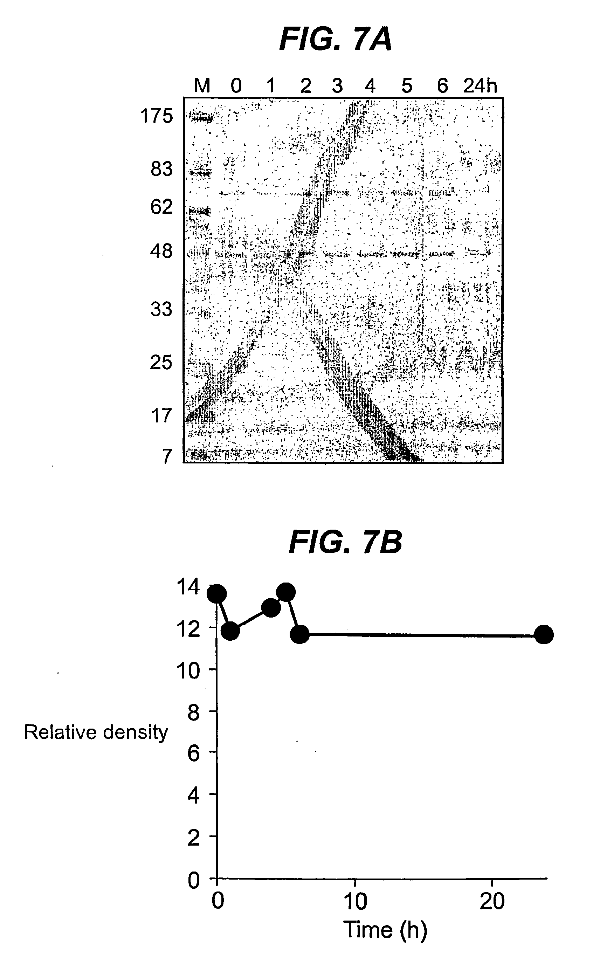

Fig 7 illustrates the retention of Sh ble fusion protein on Bleomycin derivatised microwell; (Sh Ble fusion protein clone 1 was bound to Bleomycin derivatised microwells. After washing the wells were incubated for varying times in buffer.
   A) Coomassie stained SDS PAGE showing amount of fusion protein remaining immobilized.
   B) The bands were quantified and plotted against time.)

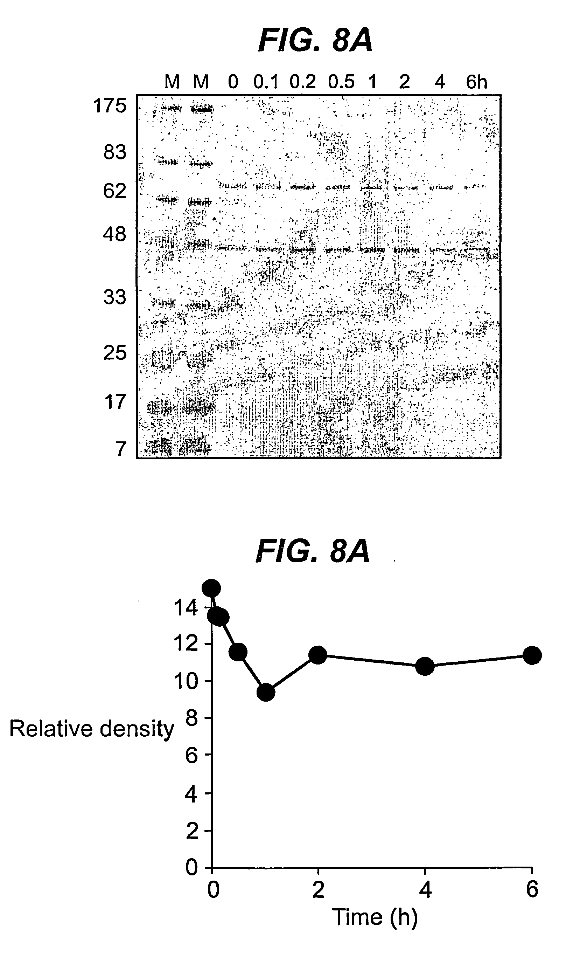

Fig 8 illustrates retention of Sh ble fusion protein on Bleomycin derivatised microwell. (Sh ble fusion protein clone 1 was bound to Bleomycin derivatised microwells. After washing the wells were incubated for varying times in buffer containing excess Bleomycin. A) Coomassie stained SDS PAGE showing amount of fusion protein remaining immobilized. B) The bands were quantified and plotted against time. and

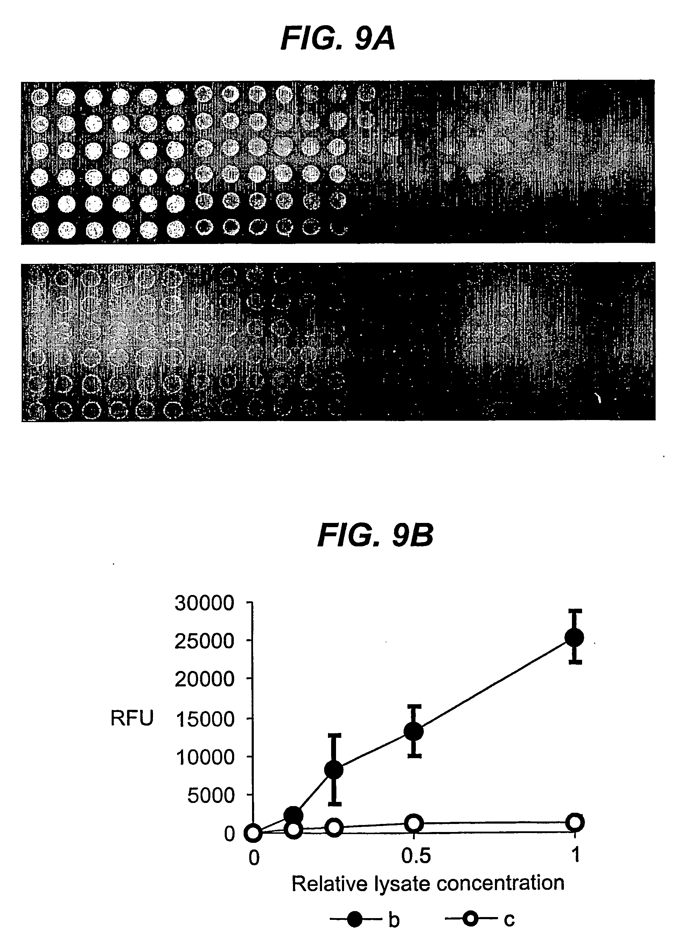

Fig 9 illustrates immobilisation of Sh ble fusion protein on a Bleomycin derivatised microarray slide. (1 µl of cleared lysates of Sh ble fusion protein clone 2 were spotted onto a bleomycin derivatised micro-array slide in the presence and absence of excess free bleomycin. A) After probing for the fusion protein using an anti Flag antibody and Cy3 labelled anti mouse secondary the Cy3 fluorophore was visualized. B) Total spot fluorescence intensity was plotted in a histogram for spots in the presence and absence of excess Bleomycin.)

### DETAILED DESCRIPTION OF THE INVENTION

### 1.0 Use of Sh ble as an affinity Tag

A cDNA library was ligated into a modified E. coli expression vector (compare to Fig 2) so that the gene could be Tagged at both the N and C terminii with a FLAG Tag at the N terminus and Sh ble, BCCP (Biotinylated domain of E. coli ACCB) and Myc Tag at the C terminus.

After transformation into XL10 Gold, clones were selected by growing on LB Agar containing 50 µg/ml Phleomycin D1.

Two Phleomycin D1 resistant clones were selected, grown in LB-amp to an OD₆₀₀ of approx 0.4 after which time the clones were induced with 100 µg /ml IPTG for 4h at 30°C. The cells were harvested by centrifugation, 4000g for 15 min at 4°C, and stored in aliquots at -20 °C to be used for future work. These are referred to as "Clone 1" and "Clone 2".

Fresh E. coli lysates were prepared prior to each experiment.

To each aliquot of cells (from 40 ml of induction culture) 300 µl of lysis buffer (200 mM Tris pH 8, 100 mM EDTA, 1 x Protease inhibitor cocktail (Roche), 10 mM mercaptoethanol, 200 µg/ml lysozyme) was added and the cells resuspended. After 15 min incubation at 4°C cells were diluted with 1.7 ml of buffer (1 x protease inhibitors, 20 mM MgCl₂, 50 µg/ml DNAse) and incubated at 4°C for a further 30 minutes. Cleared lysates were obtained after centrifugation 20000g for 10 min at 4°C.

### 1.1 Derivatisation of Sepharose with Bleomycin and its use in affinity purification.

Bleomycin A6 (2mg/ml) was coupled to a Pharmacia 1ml HiTrap NHS activated Sepharose column according to the manufacturers instruction.

After coupling the Sepharose was removed from the column and used for batchwise purification of Sh ble fusion proteins.

To 6 µl of Bleomycin-Sepharose slurry (approx 50% swollen beads) in PBS-Tween 4 µl of Clone 1 lysate (prepared as above) was added and incubated at 4°C for 30 min with shaking. After briefly centrifuging the supernatant was removed and the beads were washed three times in 10 µl of PBS-Tween. To a similarly treated batch of Bleomycin-Sepharose bound protein was eluted by incubating the beads in 10 µl of PBS-Tween containing 300 µg/ml Bleomycin A6 at 4°C for 10 min with shaking.

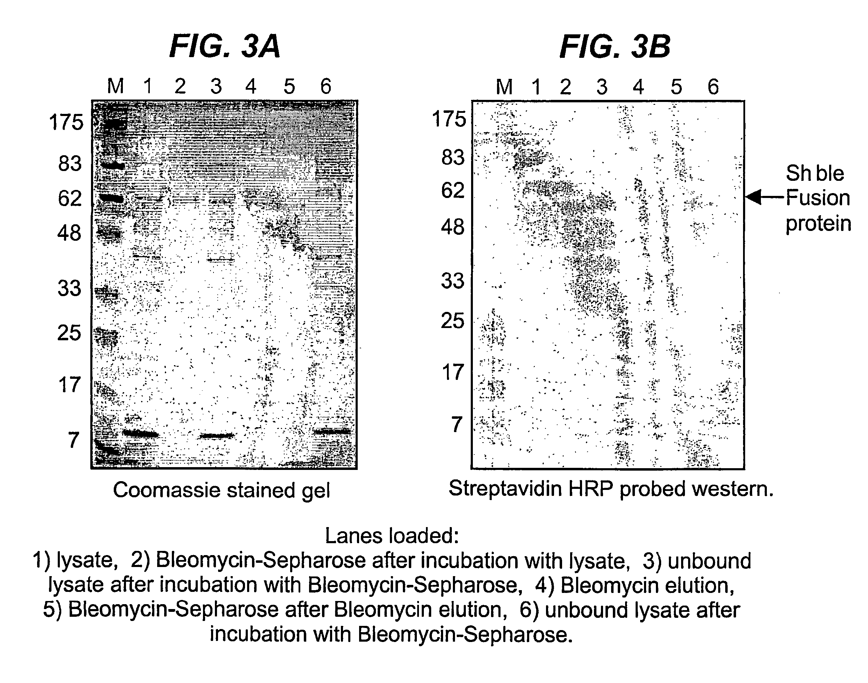

Samples were then boiled in SDS sample buffer and loaded onto two identical 10-20% Tris Glycine SDS PAGE plates. After electrophoresis one gel was stained with coomassie and the other transferred to nitrocellulose membrane and probed with streptavidin HRP before visualization of bands using DAB (Fig 3).

Referring to Fig 3 it can be clearly seen that the Sh ble fusion protein has bound to the Bleomycin sepharose on both the coomassie stained gel and Western Blot (Figure 3 A and B lanes 2). It can also be seen that the Sh ble fusion protein bound is very pure producing only one band on Coomassie stained gel. The protein has not only shown to be specifically bound and significantly depleted from the lysate of fusion protein, but also can be specifically eluted with free bleomycin (Figure 3 A and B lanes 4) thus providing evidence of both specificity of interaction and also a means of elution. However under these conditions only a proportion of the Sh ble fusion protein was eluted from the Bleomycin-Sepharose.

### 1.2 Derivatisation of Microtitre plates with Bleomycin

Microtitre 8 well strips coated with PEG with an amine reactive terminus ("protein immobilizer" plates) were purchased from Exiqon. Plates were coated with Bleomycin A6 effectively according to the manufacturers protocol. Briefly Bleomycin A6 was dissolved in 100 mM KPO₄ buffer pH 8 at a concentration of 1 mg/ml. 100 µl of Bleomycin solution was added to each well, the wells were sealed and incubated at 4°C for 18h. After which the Bleomycin solution was removed from the wells and the wells were washed with PBS-Tween for 5 minutes three times. Control wells were prepared as above with the omission of bleomycin A6.

### 1.3 Use of Bleomycin surfaces in affinity immobilization

To Bleomycin coated and control wells, 50 µl of two different Sh ble fusion protein clone lysates (Clone 1 and Clone 2 lysates prepared as above) were added in the presence and absence of 5 mg/ml bleomycin. Lysates were incubated in the wells for 30 mins at room temperature with slight agitation, after which time the lysate was aspirated from the wells and the wells were washed three times with PBS-Tween for 5 minutes each wash. Proteins were eluted from the surface by the addition of 50 µl of SDS sample loading buffer and the wells were heated on a heating block at 100°C for 10 mins. 20 µl of each sample was run on 10-20% Tris Glycine SDS PAGE. The gel was transferred to nitrocellulose membrane and probed using streptavidin HRP conjugate. The Blot was visualized using ECL (Amersham) and photographic film shown in Fig 4.

From Fig 4 it is clear that both Sh ble fusion proteins are only immobilized on Bleomycin derivatised micro wells and in addition that this interaction can be competed by preincubation of lysates with excess free bleomycin.

### 1.4 Analysis of Sh ble fusion protein purified and immobilized on Bleomycin derivatised microwells

50 µl of Cleared lysates at differing dilutions of Sh ble fusion Clone 1 prepared as above were added to Bleomycin derivatised microwells. After incubation for 40 minutes at room temperature the wells were washed three times with PBS-Tween. Protein was eluted from the wells by heating on a heating block at 100°C for 10 min in SDS sample buffer and 15 µl were electrophoresed on SDS PAGE with equivalent amounts of input lysates. After electrophoresis gels were visualised using a standard silver staining protocol.

The results are illustrated in Fig 5 which shows purification of immobilised Sh ble fusion proteins on Bleomycin coated microwells. Sh ble fusion protein captured from lysates on Bleomycin derivatised microwells was run on SDS PAGE. Input lysates were also run for comparison. Input lysates were a 2 fold dilution series, with lane 1 representing undiluted lysate and lane 7 representing a 64 fold dilution.

Figure 5 demonstrates that there is predominantly 1 major band of purified full length Sh Ble fusion protein captured on microwells. It is probable that other minor bands seen represent proteolysis products of the Sh ble fusion as shown by comparison to a similar experiment with western blotting shown in Fig 4.

### 1.5 Rate of binding of Sh ble fusion protein to Bleomycin derivatised microwells

50 µl of cleared lysate of Sh ble fusion Clone 1 were added to Bleomycin derivatised microwells. After 5, 10, 20, 30, 40, 50 and 60 minutes of incubation at room temperature the wells were washed three times with PBS-Tween. Protein was eluted from the wells by heating on a heating block at 100°C for 10 min in SDS sample buffer and 15 µl were electrophoresed on SDS PAGE. After electrophoresis gels were visualised using a standard silver staining protocol (Fig 6 A). The Sh ble bands on the gel image were quantified and average density was plotted (Fig 6 B). These data show that most protein is immobilized after approximately 40 minutes of incubation.

### 1.6 Stability of Sh ble fusion protein interaction with Bleomycin derivatised microwells.

50 µl of cleared lysate of Sh Ble fusion Clone 1 were added to Bleomycin derivatised microwells. After incubation for 1h at room temperature the wells were washed three times with PBS-Tween. After washing wells were filled with PBS-Tween and incubated for the desired time to generate a time course. This was also repeated with a duplicate set of wells, which after washing the wells were incubated in 0.5mM Bleomycin in PBS-Tween. After these time courses, protein was eluted from the wells by heating on a heating block at 100°C for 10 min in SDS sample buffer and 15 µl were electrophoresed on SDS PAGE with equivalent amounts of input lysates. After electrophoresis gels were visualised using coomassie stain (Figs. 7A & 8A).

The Sh ble fusion protein bands on the gel image were quantified and average density was plotted (Figs. 7B & 8B).

Immobilised Sh ble fusion protein remained very largely bound to the Bleomycin derivatised microwell after incubation for up to 24 h in PBS-Tween (Fig. 7). The inclusion of excess free Bleomycin in the buffer was anticipated to increase the rate of fusion protein dissociation from the microwell, however even in the presence of excess free Bleomycin (Fig. 8) little loss of immobilized Sh ble fusion protein was observed.

### 1.7 Use of Sh ble fusion for immobilization of proteins on microarrays.

Exiqon Euray slides were used in this study and have a surface coating that is essentially that found in the microtitre plate wells used previously, where a PEG molecule with an amine reactive terminus is used to coat the slide. Derivatisation of these slides was performed as for the microtitre plate wells with 400 µl of 3 mg/ml Bleomycin A6 applied to the surface under a microscope slide hybridization chamber.

Cleared lysates of Sh ble fusion protein clone 1 were diluted 1 in 2, 1 in 4 and 1 in 8 with 100 mM KPO₄ buffer. The lysates were transferred to a 384 well microtitre plate for microarraying. A six by six pattern of the lysates was microarrayed onto one slide derivatised with bleomycin and one that had been similarly coated with ethanolamine. Microarraying was achieved using a Genetix Qarray robot equipped with 300 micron solid pins. The microarrays were incubated in a humid chamber for 1 h at room temperature. After washing in PBS-Tween the slides were probed with a mouse anti FLAG antibody followed by a Cy3 labelled anti mouse IgG antibody. After drying in a stream of nitrogen, slides were visualized using an Affymetrix 428 array scanner equipped with laser and filter appropriate for detecting the Cy3 Fluorophore (Fig. 9A). The total spot fluorescence was quantified and averages of the identical spots were taken and plotted (Fig. 9B).

The data in Figure 9 demonstrate that a Sh ble fusion protein is immobilized specifically on a bleomycin derivatised microscope slide. This indicates that Sh ble is suitable for use as an affinity tag for the immobilization of proteins in a microarray format.

## Claims

1. A method of immobilising a protein to a surface, wherein the protein is provided to the surface as a Ble fusion protein and the surface is a surface derivatised with an antibiotic from the bleomycin family.

2. The method as claimed in claim 1, wherein the Ble within a Ble fusion protein is also used as an expression and folding marker,

3. The method as claimed in claim 1 or claim 2 wherein the Ble fusion protein is the expression product of a Sh ble, Tn5 ble or Sa ble gene.

4. A method as claimed in claim 1 wherein the antibiotic from the bleomycin family is selected from the group consisting of bleomycin, phleomycin, tallysomycin, pepleomycin and Phleomycin D 1.

5. A method as claimed in claim 4 wherein the antibiotic from the bleomycin family is selected from the group consisting of bleomycin A2, bleomycin A5, bleomycin A6, bleomycin B2 or Phleomycin D 1,

6. A method as claimed in any of claims 1-5 wherein a functional group on the antibiotic is used to link it to the surface.

7. A method as claimed in claim 6 wherein an amine group present on the antibiotic is used to couple the antibiotic to the surface.

8. A method as claimed in claim 7 wherein the antibiotic is coupled to a polyethyleneglycol (PEG) derivitized surface via an amine group.

9. A method as claimed in any of claims 1-8 wherein the surface is the surface of an array, a microtitre plate, a slide or a bead.

10. A method as claimed in claim 9 wherein the array is a microarray.

11. A method as claimed in claim 10 wherein the array is a MALDI array.

12. A method as claimed in claim 9 which further comprises removing the Ble fusion protein from the surface.

13. A probe, **characterized in that** it has a target surface comprising an array having a plurality of discrete target areas presenting one or more analyte capture moieties comprising an antibiotic from the bleomycin family.

14. A probe as claimed in claim 13 wherein the antibiotic is provided on the target surface at a high surface density such that one would expect on average that one Sh ble dimer would be bound by two bleomycin molecules.

15. A probe as claimed in claim 14 wherein the capture moieties have an affinity for the moiety they are intended to capture of less than 400nM, and preferably less than 200nM.

16. A probe as claimed in any of claims 13-15 wherein the antibiotic from the bleomycin family is selected from the group consisting of bleomycin, phleomycin, tallysomycin, pepleomycin and Phleomycin D1.

17. A probe as claimed in claim 16 wherein the antibiotic from the bleomycin family is selected from the group consisting of bleomycin A2, bleomycin A5, bleomycin A6, bleomycin B2 or Phleomycin D1,

18. A purification medium comprising a target surface presenting one or more analyte capture moieties comprising an antibiotic from the bleomycin family.

19. A purification medium as claimed in claim 18 which is a bead.

20. A purification medium as claimed in claim 18 or 19 wherein the antibiotic is provided on the target surface at a low surface density such that one would expect on average that one Sh ble dimer would be bound by one bleomycin molecule.

21. A purification medium as claimed in claim 20 wherein the capture moieties have an affinity for the moiety they are intended to capture of greater than 400nM and preferably greater than 500nM.

22. A purification medium as claimed in any of claims 18-21 wherein the antibiotic from the bleomycin family is selected from the group consisting of bleomycin, phleomycin, tallysomycin, pepleomycin and Phleomycin D1.

23. A purification medium as claimed in any of claims 18-22 wherein the antibiotic from the bleomycin family is selected from the group consisting of bleomycin A2, bleomycin A5, bleomycin A6, bleomycin B2 or Phleomycin D1.

24. A purification medium as claimed in any of claims 18-23 wherein the antibiotic is bound to the surface via a flexible linker molecule.

25. A purification medium as claimed in claim 24 wherein the flexible linker molecule is a polyethylene glycol (PEG).

26. A method for generating soluble forms of an insoluble protein comprising: i) generating a library of protein variants; and ii) selecting colonies for the presence of a soluble protein by expressing the protein as a Ble fusion protein and selecting on an antibiotic from the bleomycin family; and (iii) growing the selected colonies, lysing them and binding the fusion protein to a surface derivatised with an antibiotic from the bleomycin family.

27. The method of claim 26, further comprising washing the tagged surface to remove non-bound materials.

28. A method of purifying a Ble fusion protein from a crude extract comprising the step of immobilising it on a surface via an antibiotic from the bleomycin family and optionally releasing it there from.

29. A method of identifying the cellular localisation of a protein comprising i) expressing the protein as a Ble fusion protein in a cell, ii) introducing a labelled antibiotic from the bleomycin family into the cell, and iii) detecting the labelled antibiotic.

30. A method as claimed in claim 29 wherein the antibiotic is an antibiotic from the bleomycin family **characterized in that** it is tagged with a marker.

31. A method as claimed in claim 30 wherein the marker is a visual marker.

32. A method as claimed in claim 31 wherein the visual marker is a fluorescent marker.

33. A method as claimed in claim 32 wherein the fluorescent marker is selected from NHS-activated fluorescein, Cy3, Cy5, or Rhodamine.

34. A kit for the production of an array comprising a ble vector and a surface derivatised with an antibiotic from the bleomycin family or the components for making said derivatised surface.

## Patentansprüche

1. Verfahren zum Immobilisieren eines Proteins an einer Oberfläche, wobei das Protein an die Oberfläche als ein Ble Fusionsprotein bereitgestellt wird und die Oberfläche eine Oberfläche ist, die mit einem Antibiotikum aus der Bleomycin Familie derivatisiert wurde.

2. Verfahren wie in Anspruch 1 beansprucht, wobei das Ble innerhalb eines Ble Fusionsproteins auch als ein Expressions- und Faltungsmarker verwendet wird.

3. Verfahren wie in Anspruch 1 oder Anspruch 2 beansprucht, wobei das Ble Fusionsprotein ein Expressionsprodukt eines Sh ble, Tn5 ble oder Sa ble Gens ist.

4. Verfahren wie in Anspruch 1 beansprucht, wobei das Antibiotikum aus der Bleomycin Familie ausgewählt ist aus der Gruppe, die aus Bleomycin, Phleomycin, Tallysomycin, Pepleomycin und Phleomycin D1 besteht.

5. Verfahren wie in Anspruch 4 beansprucht, wobei das Antibiotikum aus der Bleomycin Familie ausgewählt ist aus der Gruppe, die aus Bleomycin A2, Bleomycin A5, Bleomycin A6, Bleomycin B2 oder Phleomycin D1 besteht.

6. Verfahren wie in einem der Ansprüche 1-5 beansprucht, wobei eine funktionelle Gruppe an dem Antibiotikum verwendet wird, um es mit der Oberfläche zu verknüpfen.

7. Verfahren wie in Anspruch 6 beansprucht, wobei eine Amingruppe, die an dem Antibiotikum anwesend ist, verwendet wird, um das Antibiotikum an die Oberfläche zu koppeln.

8. Verfahren wie in Anspruch 7 beansprucht, wobei das Antibiotikum an einer Polyethylenglykol (PEG) derivatisierten Oberfläche über eine Amingruppe gekoppelt ist.

9. Verfahren wie in einem der Ansprüche 1-8 beansprucht, wobei die Oberfläche die Oberfläche eines Arrays, einer Mikrotiterplatte, eines Objektträgers oder eines Kügelchens ist.

10. Verfahren wie in Anspruch 9 beansprucht, wobei der Array ein Mikroarray ist.

11. Verfahren wie in Anspruch 10 beansprucht, wobei der Array ein MALDI Array ist.

12. Verfahren wie in Anspruch 9 beansprucht, das weiterhin das Entfernen des Ble Fusionsproteins von der Oberfläche umfasst.

13. Sonde, **dadurch gekennzeichnet, dass** sie eine Zieloberfläche aufweist, die einen Array mit einer Vielzahl von einzelnen Zielbereichen umfasst, die einen oder mehrere Analyt-Fängerrest/e darstellen, der/die ein Antibiotikum aus der Bleomycin Familie umfasst/umfassen.

14. Sonde wie in Anspruch 13 beansprucht, wobei das Antibiotikum an der Zieloberfläche mit einer hohen Oberflächendichte bereitgestellt wird, so dass man erwarten würde, dass durchschnittlich ein Sh ble Dimer von zwei Bleomycin Molekülen gebunden wird.

15. Sonde wie in Anspruch 14 beansprucht, wobei die Fängerreste eine Affinität für den Rest, den sie zu fangen beabsichtigen, von weniger als 400 nM, und vorzugsweise weniger als 200 nM aufweisen.

16. Sonde wie in einem der Ansprüche 13-15 beansprucht, wobei das Antibiotikum aus der Bleomycin Familie ausgewählt ist aus der Gruppe, die aus Bleomycin, Phleomycin, Tallysomycin, Pepleomycin und Phleomycin D1 besteht.

17. Sonde wie in Anspruch 16 beansprucht, wobei das Antibiotikum aus der Bleomycin Familie ausgewählt ist aus der Gruppe, die aus Bleomycin A2, Bleomycin A5, Bleomycin A6, Bleomycin B2 oder Phleomycin D1 besteht.

18. Reinigungsmedium umfassend eine Zieloberfläche, die einen oder mehrere Analyt-Fängerrest/e präsentiert, der/die ein Antibiotikum aus der Bleomycin Familie umfasst/umfassen.

19. Reinigungsmedium wie in Anspruch 18 beansprucht, das ein Kügelchen ist.

20. Reinigungsmedium wie in Anspruch 18 oder 19 beansprucht, wobei das Antibiotikum an der Zieloberfläche in einer geringen Oberflächendichte bereitgestellt wird, so dass man erwarten würde, dass durchschnittlich ein Sh ble Dimer von einem Bleomycin Molekül gebunden wird.

21. Reinigungsmedium wie in Anspruch 20 beansprucht, wobei die Fängereinheiten eine Affinität für den Rest, den sie zu fangen beabsichtigen, von größer als 400 nM und vorzugsweise größer als 500 nM aufweisen.

22. Reinigungsmedium wie in einem der Ansprüche 18-21 beansprucht, wobei das Antibiotikum aus der Bleomycin Familie ausgewählt ist aus der Gruppe, die aus Bleomycin, Phleomycin, Tallysomycin, Pepleomycin und Phleomycin D1 besteht.

23. Reinigungsmedium wie in einem der Ansprüche 18-22 beansprucht, wobei das Antibiotikum aus der Bleomycin Familie ausgewählt ist aus der Gruppe, die aus Bleomycin A2, Bleomycin A5, Bleomycin A6, Bleomycin B2 oder Phleomycin D1 besteht.

24. Reinigungsmedium wie in einem der Ansprüche 18-23 beansprucht, wobei das Antibiotikum an die Oberfläche über ein flexibles Linkermolekül gebunden ist.

25. Reinigungsmedium wie in Anspruch 24 beansprucht, wobei das flexible Linkermolekül ein Polyethylenglykol (PEG) ist.

26. Verfahren zum Herstellen löslicher Formen eines unlöslichen Proteins umfassend: i) Herstellen einer Bibliothek von Proteinvarianten; und ii) Auswählen von Kolonien hinsichtlich der Anwesenheit eines löslichen Proteins durch Exprimieren des Proteins als ein Ble Fusionsprotein und Auswählen eines Antibiotikums aus der Bleomycin Familie; und iii) Anziehen der ausgewählten Kolonien, ihr Lysieren und Binden des Fusionsproteins an eine Oberfläche, die mit einem Antibiotikum aus der Bleomycin Familie derivatisiert ist.

27. Verfahren nach Anspruch 26, weiterhin umfassend Waschen der markierten Oberfläche, um nichtgebundene Materialien zu entfernen.

28. Verfahren zum Reinigen eines Ble Fusionsproteins aus einem Rohextrakt umfassend den Schritt seines Immobilisierens auf einer Oberfläche über ein Antibiotikum aus der Bleomycin Familie und wahlweise sein Freisetzen davon.

29. Verfahren zum Identifizieren der zellulären Lokalisation eines Proteins umfassend i) Exprimieren des Proteins als ein Ble Fusionsprotein in einer Zelle, ii) Einführen eines markierten Antibiotikums aus der Bleomycin Familie in die Zelle, und iii) Nachweisen des markierten Antibiotikums.

30. Verfahren wie in Anspruch 29 beansprucht, wobei das Antibiotikum ein Antibiotikum aus der Bleomycin Familie ist, das **dadurch gekennzeichnet ist, dass** es mit einem Marker markiert ist.

31. Verfahren wie in Anspruch 30 beansprucht, wobei der Marker ein visueller Marker ist.

32. Verfahren wie in Anspruch 31 beansprucht, wobei der visuelle Marker ein Fluoreszenzmarker ist.

33. Verfahren wie in Anspruch 32 beansprucht, wobei der Fluoreszenzmarker ausgewählt ist unter NHS-aktiviertem Fluorescein, Cy3, Cy5 oder Rhodamin.

34. Kit für die Herstellung eines Arrays umfassend einen ble Vektor und eine Oberfläche, die mit einem Antibiotikum aus der Bleomycin Familie oder den Bestandteilen zur Herstellung der derivatisierten Oberfläche derivatisiert ist.

## Revendications

1. Procédé d'immobilisation d'une protéine sur une surface, dans lequel la protéine est mise en oeuvre sur la surface en tant que protéine de fusion de Ble et la surface est une surface modifiée avec un antibiotique de la famille de la bléomycine.

2. Procédé selon la revendication 1, dans lequel la Ble au sein d'une protéine de fusion de Ble est également utilisée comme marqueur d'expression et de repliement.

3. Procédé selon la revendication 1 ou 1a revendication 2, dans lequel la protéine de fusion de Ble est le produit d'expression d'un gène Sh ble, Tn5 ble ou Sa ble.

4. Procédé selon la revendication 1, dans lequel l'antibiotique de la famille de la bléomycine est choisi dans le groupe constitué par la bléomycine, la phléomycine, la tallysomycine, la pépléomycine et la phléomycine D1.

5. Procédé selon la revendication 4, dans lequel l'antibiotique de la famille de la bléomycine est choisi dans le groupe constitué par la bléomycine A2, la bléomycine A5, la bléomycine A6, la bléomycine B2 et la phléomycine D1.

6. Procédé selon l'une quelconque des revendications 1 à 5, dans lequel on utilise un groupe fonctionnel sur l'antibiotique pour le fixer à la surface.

7. Procédé selon la revendication 6, dans lequel on utilise un groupe amine présent sur l'antibiotique pour coupler l'antibiotique à la surface.

8. Procédé selon la revendication 7, dans lequel l'antibiotique est couplé à une surface modifiée avec du polyéthylène glycol (PEG) via un groupe amine.

9. Procédé selon l'une quelconque des revendications 1 à 8, dans lequel la surface est la surface d'un réseau, d'une plaque de microtitrage, d'une lame ou d'une bille.

10. Procédé selon la revendication 9, dans lequel le réseau est un microréseau.

11. Procédé selon la revendication 10, dans lequel le réseau est un réseau MALDI.

12. Procédé selon la revendication 9, qui comprend en outre l'élimination de la protéine de fusion Ble de la surface.

13. Sonde, **caractérisée en ce qu'**elle présente une surface cible comprenant un réseau ayant une pluralité de zones cibles discrètes présentant un ou plusieurs fragments de capture d'analytes, comprenant un antibiotique de la famille de la bléomycine.

14. Sonde selon la revendication 13, dans laquelle l'antibiotique est mis en oeuvre sur la surface cible à une densité de surface élevée, de telle sorte que l'on s'attende en moyenne à ce qu'un dimère de Sh ble soit fixé par deux molécules de bléomycine.

15. Sonde selon la revendication 14, dans laquelle les fragments de capture ont une affinité pour le fragment qu'ils sont censés capturer, inférieure à 400 nM et de préférence, inférieure à 200 nM.

16. Sonde selon l'une quelconque des revendications 13 à 15, dans laquelle l'antibiotique de la famille de la bléomycine est choisi dans le groupe constitué par la bléomycine, la phléomycine, la tallysomycine, la pépléomycine et la phléomycine D1.

17. Sonde selon la revendication 16, dans laquelle l'antibiotique de la famille de la bléomycine est choisi dans le groupe constitué par la bléomycine A2, la bléomycine A5, la bléomycine A6, la bléomycine B2 et la phléomycine D1.

18. Milieu de purification comprenant une surface cible présentant un ou plusieurs fragments de capture d'analytes, comprenant un antibiotique de la famille de la bléomycine.

19. Milieu de purification selon la revendication 18, qui est une bille.

20. Milieu de purification selon la revendication 18 ou 19, dans lequel l'antibiotique est mis en oeuvre sur la surface cible à une faible densité de surface, de telle sorte que l'on s'attende en moyenne à ce qu'un dimère de Sh ble soit fixé par une molécule de bléomycine.

21. Milieu de purification selon la revendication 20, dans lequel les fragments de capture ont une affinité pour le fragment qu'ils sont censés piéger, supérieure à 400 nM et, de préférence, supérieure à 500 nM.

22. Milieu de purification selon l'une quelconque des revendications 18 à 21, dans lequel l'antibiotique de la famille de la bléomycine est choisi dans le groupe constitué par la bléomycine, la phléomycine, la tallysomycine, la pépléomycine et la phléomycine D1.

23. Milieu de purification selon l'une quelconque des revendications 18 à 22, dans lequel l'antibiotique de la famille de la bléomycine est choisi dans le groupe constitué par la bléomycine A2, la bléomycine A5, la bléomycine A6, la bléomycine B2 et la phléomycine D1.

24. Milieu de purification selon l'une quelconque des revendications 18 à 23, dans lequel l'antibiotique est fixé à la surface via une molécule de liaison flexible.

25. Milieu de purification selon la revendication 24, dans lequel la molécule de liaison flexible est une molécule de polyéthylène glycol (PEG).

26. Procédé pour générer des formes solubles d'une protéine insoluble comprenant i) la génération d'une bibliothèque de variants de la protéine et ii) la sélection de colonies pour la présence d'une protéine soluble par expression de la protéine en tant que protéine de fusion de Ble et la sélection sur un antibiotique de la famille de la bléomycine et iii) la culture des colonies sélectionnées, leur lyse et la liaison de la protéine de fusion à une surface modifiée avec un antibiotique de la famille de la bléomycine.

27. Procédé selon la revendication 26, comprenant en outre le lavage de la surface marquée pour éliminer les substances non fixées.

28. Procédé de purification d'une protéine de fusion de Ble à partir d'un extrait brut, comprenant l'étape d'immobilisation de celle-ci sur une surface via un antibiotique de la famille de la bléomycine et éventuellement la libération de celle-ci à partir de la surface.

29. Procédé d'identification de la localisation cellulaire d'une protéine comprenant i) l'expression de la protéine en tant que protéine de fusion de Ble dans une cellule, ii) l'introduction dans la cellule d'un antibiotique marqué de la famille de la bléomycine et iii) la détection de l'antibiotique marqué.

30. Procédé selon la revendication 29, dans lequel l'antibiotique est un antibiotique de la famille de la bléomycine **caractérisé en ce qu'**il est marqué à l'aide d'un marqueur.

31. Procédé selon la revendication 30, dans lequel le marqueur est un marqueur visuel.

32. Procédé selon la revendication 31, dans lequel le marqueur visuel est un marqueur fluorescent.

33. Procédé selon la revendication 32, dans lequel le marqueur fluorescent est choisi parmi la fluorescéine activée par NHS, le Cy3, le Cy5 ou la rhodamine.

34. Kit pour la production d'un réseau comprenant un vecteur de ble et une surface modifiée avec un antibiotique de la famille de la bléomycine ou les composants pour préparer ladite surface modifiée.
